(19) 

Europäisches Patentamt
European Patent Office
Office européen des brevets

(11) **EP 4 458 318 A1**

(12) **EUROPEAN PATENT APPLICATION**
published in accordance with Art. 153(4) EPC

(43) Date of publication:
**06.11.2024 Bulletin 2024/45**

(21) Application number: **22916167.4**

(22) Date of filing: **28.12.2022**

(51) International Patent Classification (IPC):
***A61C 13/083*** (2006.01)    ***C04B 35/488*** (2006.01)

(52) Cooperative Patent Classification (CPC):
**A61C 13/083; C04B 35/488**

(86) International application number:
**PCT/JP2022/048500**

(87) International publication number:
**WO 2023/127945 (06.07.2023 Gazette 2023/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.12.2021 JP 2021215270**

(71) Applicant: **Kuraray Noritake Dental Inc.**
**Kurashiki-shi, Okayama 710-0801 (JP)**

(72) Inventors:
- **KATO, Shinichiro**
  **Miyoshi-shi, Aichi 470-0293 (JP)**
- **MATSUMOTO, Atsushi**
  **Miyoshi-shi, Aichi 470-0293 (JP)**

(74) Representative: **D Young & Co LLP**
**3 Noble Street**
**London EC2V 7BQ (GB)**

(54) **ZIRCONIA CALCINED BODY SUITABLE FOR DENTAL USE**

(57)    The present invention provides a zirconia pre-sintered body having suitable shades, translucency, and strength. The present invention relates to a zirconia pre-sintered body comprising a layered structure of at least three layers containing zirconia and a stabilizer capable of preventing a phase transformation of zirconia, the layered structure including at least two layers that differ from one another in the content of the stabilizer relative to the total mole of the zirconia and the stabilizer, the layered structure including at least two layers having substantially the same stabilizer content relative to the total mole of the zirconia and the stabilizer, the zirconia pre-sintered body containing a coloring component in all of the layers having substantially the same stabilizer content, and the layers having substantially the same stabilizer content differing from one another in the composition of the coloring component.

FIG.1

**Description**

TECHNICAL FIELD

The present invention relates to a zirconia pre-sintered body suitable for dental use, a method of production thereof, and to a method for producing a dental product.

BACKGROUND ART

Zirconia is a compound that undergoes a phase transformation between crystal systems. In this connection, partially-stabilized zirconia (PSZ) and fully-stabilized zirconia, which exist as a solid solution of zirconia with stabilizers such as yttria (yttrium oxide; $Y_2O_3$) to prevent such phase transformations, are used in a wide variety of fields.
In the field of dentistry, zirconia materials are used as materials of frames due to their high-strength characteristics. Moreover, recent improvements in the translucency of zirconia materials have led to increased production of zirconia-only dental prostheses. Such zirconia materials are proposed in, for example, Patent Literatures 1 to 3.
Patent Literature 1 discloses a zirconia sintered body with a gradation of shades from the incisal region toward the cervical region
Patent Literature 2 discloses a zirconia sintered body with layers having different yttria contents, and the yttria content is decreased from the incisal region toward the cervical region to produce appropriate translucency as a dental prosthesis.
Patent Literature 3 discloses a zirconia sintered body containing a pigment and in which the yttria content is different from layer to layer, and the yttria content is decreased from the incisal region toward the cervical region to produce appropriate translucency as a dental prosthesis.

CITATION LIST

Patent Literature

Patent Literature 1: WO2019/131782
Patent Literature 2: US Patent Application Publication Number 2013/0221554
Patent Literature 3: US Patent Application Publication Number 2016/0120765

SUMMARY OF INVENTION

Technical Problem

As discussed above, there is an increasing trend to more conveniently prepare zirconia dental prostheses at the dental clinic, and a need exists to develop excellent aesthetic quality while maintaining the strength with zirconia alone.
The natural teeth structure comprises enamel, dentin, and pulp, with the dentin situated beneath the enamel, and the pulp located inside. Because the aesthetics (appearance) of natural teeth are produced through the intricate interplay of shades and translucency in different parts of teeth, traditional methods have failed to faithfully reproduce this aesthetic quality with zirconia alone.
The zirconia pre-sintered body described in Patent Literature 1 shows a gradation of shades after sintering, and appears to exhibit a certain aesthetic quality. However, the yttria content is the same throughout from the cervical region to the incisal region, failing to satisfy the levels of translucency and shade required for the incisal region while providing the strength and shade needed for the cervical region.
The zirconia described in Patent Literatures 2 and 3 possesses layers differing in yttria content, suggesting that the translucency and strength may be appropriate as dental prostheses. However, because each layer has a different yttria content, there is a challenge in addressing the complexity of translucency and shade gradations that enables faithful reproduction of natural teeth, where there is an intricate interplay of shades and translucency in different parts of teeth as noted above.
There accordingly is a need for zirconia pre-sintered bodies that exhibit suitable strength for dental applications as sintered bodies following firing while possessing the complex shades and translucency of natural teeth.
An object of the present invention is to provide a zirconia pre-sintered body having suitable shades, translucency, and strength.
Another object of the present invention is to provide a zirconia pre-sintered body satisfying the translucency and shade required for the incisal region while possessing the strength and shade needed for the cervical region.

Solution to Problem

The present inventors conducted intensive studies to find a solution to the problems discussed above, and found that these issues can be solved with a zirconia pre-sintered body having the appropriately adjusted content of stabilizer and coloring component in each layer. This led to the completion of the present invention after further examinations.

Specifically, the present invention includes the following.

[1] A zirconia pre-sintered body comprising a layered structure of at least three layers containing zirconia and a stabilizer capable of preventing a phase transformation of zirconia,

the layered structure including at least two layers that differ from one another in the content of the stabilizer relative to the total mole of the zirconia and the stabilizer,
the layered structure including at least two layers having substantially the same stabilizer content relative to the total mole of the zirconia and the stabilizer,
the zirconia pre-sintered body containing a coloring component in all of the layers having substantially the same stabilizer content, and the layers having substantially the same stabilizer content differing from one another in the composition of the coloring component.

[2] The zirconia pre-sintered body according to [1], wherein the layers having substantially the same stabilizer content are adjacent to one another.
[3] The zirconia pre-sintered body according to [1] or [2], wherein the coloring component comprises an oxide of at least one element selected from the group consisting of Ti, V, Cr, Mn, Fe, Co, Ni, Pr, Tb, and Er; or $(Zr,V)O_2$.
[4] The zirconia pre-sintered body according to any one of [1] to [3], wherein the layered structure includes only one layer having the highest stabilizer content.
[5] The zirconia pre-sintered body according to any one of [1] to [4], wherein the layer having the highest stabilizer content does not contain a coloring component.
[6] The zirconia pre-sintered body according to any one of [1] to [4], wherein the layer having the highest stabilizer content contains a coloring component.
[7] The zirconia pre-sintered body according to [6], wherein the coloring component contained in the layer having the highest stabilizer content differs in composition from the coloring components of the other layers.
[8] The zirconia pre-sintered body according to any one of [1] to [7], wherein, in the layers that differ from one another in the content of the stabilizer, the stabilizer content relative to the total mole of the zirconia and the stabilizer shows an unchanging pattern of increase or decrease from one end to the other end of the zirconia pre-sintered body on a straight line extending along a first direction from one end to the other end of the zirconia pre-sintered body.
[9] The zirconia pre-sintered body according to any one of [1] to [8], wherein the stabilizer is yttria.
[10] The zirconia pre-sintered body according to [9], wherein, in the layers that differ from one another in the content of yttria, the stabilizer content relative to the total mole of the zirconia and the stabilizer shows an unchanging pattern of increase or decrease from one end to the other end of the zirconia pre-sintered body on a straight line extending along a first direction from one end to the other end of the zirconia pre-sintered body, and wherein the yttria content relative to the total mole of the zirconia and the yttria is 3.5 mol% or more and 6.5 mol% or less in the layer including said one end, and is 2.5 mol% or more and less than 4.5 mol% in the layer including said other end.
[11] The zirconia pre-sintered body according to [10], wherein, in the layers that differ from one another in the content of yttria, the stabilizer content relative to the total mole of the zirconia and the stabilizer shows an unchanging pattern of increase or decrease from one end to the other end of the zirconia pre-sintered body on a straight line extending along a first direction from one end to the other end of the zirconia pre-sintered body, and wherein the difference in yttria content between the layer including said one end and the layer including said other end is 3.0 mol% or less relative to the total mole of the zirconia and the yttria.
[12] The zirconia pre-sintered body according to any one of [1] to [11], which has a color difference $\Delta E_2^*$ of 0.3 or more and 6.0 or less in the formula (4) below representing the difference in shade between a first layer and a second layer of when the shade $(L_1^*, a_1^*, b_1^*)$ of the first layer and the shade $(L_2^*, a_2^*, b_2^*)$ of the second layer are compared for two of the layers having substantially the same stabilizer content in a sintered body fabricated by firing the zirconia pre-sintered body at an adequate sintering temperature for 120 minutes,

$$\Delta E_2^* = \{(L_2^* - L_1^*)^2 + (a_2^* - a_1^*)^2 + (b_2^* - b_1^*)^2\}^{1/2} \qquad (4)$$

where ($L_1^*$, $a_1^*$, $b_1^*$) represents the shade of the first layer in the layers having substantially the same stabilizer content, and ($L_2^*$, $a_2^*$, $b_2^*$) represents the shade of the second layer in the layers having substantially the same stabilizer content.

[13] The zirconia pre-sintered body according to any one of [1] to [12], which shows a changing pattern of increase or decrease in post-sintering L* value according to L*a*b* color system from a first point toward a second point on a straight line connecting the first point and the second point when the first point falling within an interval from one end of the zirconia pre-sintered body to 25% of the entire length on a straight line extending along a first direction from said one end to the other end of the zirconia pre-sintered body has post-sintering (L*, a*, b*) of (L1, a1, b1) according to L*a*b* color system, and the second point falling within an interval from said other end to 25% of the entire length on a straight line extending along the first direction has post-sintering (L*, a*, b*) of (L2, a2, b2) according to L*a*b* color system.

[14] The zirconia pre-sintered body according to [13], wherein L1 < L3 > L2 when a third point falling between the first point and the second point on a straight line connecting the first point and the second point has post-sintering (L*, a*, b*) of (L3, a3, b3) according to L*a*b* color system.

[15] A method for producing a zirconia sintered body, comprising sintering a zirconia pre-sintered body of any one of [1] to [14] at a highest sintering temperature of 1,400°C to 1,650°C.

[16] A method for producing a dental product, comprising sintering a zirconia pre-sintered body of any one of [1] to [14] after milling.

[17] The method according to [16], wherein the milling uses a CAD/CAM system.

Advantageous Effects of Invention

According to the present invention, a zirconia pre-sintered body can be provided that has suitable shades, translucency, and strength.

According to the present invention, a zirconia pre-sintered body can be provided that exhibits suitable strength for dental applications as a sintered body following firing while possessing the complex shades and translucency of natural teeth.

According to the present invention, a zirconia pre-sintered body can be provided that satisfies the translucency and shade required for the incisal region while possessing the strength and shade needed for the cervical region.

BRIEF DESCRIPTION OF DRAWINGS

[FIG. 1] A schematic view of a zirconia pre-sintered body.

[FIG. 2] A photograph showing the appearance of zirconia sintered bodies in relation to determination of an adequate sintering temperature.

DESCRIPTION OF EMBODIMENTS

A zirconia pre-sintered body of the present invention comprises a layered structure of at least three layers containing zirconia and a stabilizer capable of preventing a phase transformation of zirconia (hereinafter, also referred to simply as "stabilizer"),

the layered structure including at least two layers that differ from one another in the content of the stabilizer relative to the total mole of the zirconia and the stabilizer (hereinafter, also referred to simply as "layers having different stabilizer contents"),

the layered structure including at least two layers having substantially the same stabilizer content relative to the total mole of the zirconia and the stabilizer (hereinafter, also referred to simply as "layers having substantially the same stabilizer content"),

the zirconia pre-sintered body containing a coloring component in all of the layers having substantially the same stabilizer content, and the layers having substantially the same stabilizer content differing from one another in the composition of the coloring component.

A zirconia pre-sintered body of the present invention is described below.

The zirconia pre-sintered body can be a precursor (intermediate product) of a zirconia sintered body.

In this specification, "zirconia pre-sintered body" refers to, for example, a state where zirconia particles (powder) are not fully sintered. The zirconia pre-sintered body may have a block shape (discotic or cuboidal).

The zirconia pre-sintered body has a density of preferably 2.7 g/cm$^3$ or more.

The zirconia pre-sintered body has a density of preferably 4.0 g/cm$^3$ or less, more preferably 3.8 g/cm$^3$ or less, even more preferably 3.6 g/cm$^3$ or less. The zirconia pre-sintered body is easily workable when the density falls within these ranges.

In the present specification, the upper limits and lower limits of numeric ranges (for example, ranges of contents of components, ranges of values calculated from components, and ranges of physical properties) can be combined appropriately.

A zirconia pre-sintered body of the present invention comprises a layered structure of at least three layers, and each layer contains zirconia and a stabilizer capable of preventing a phase transformation of zirconia. The stabilizer is preferably one capable of forming partially-stabilized zirconia.

Examples of the stabilizer include oxides such as calcium oxide (CaO), magnesium oxide (MgO), yttria (Y$_2$O$_3$), cerium oxide (CeO$_2$), scandium oxide (Sc$_2$O$_3$), niobium oxide (Nb$_2$O$_5$), lanthanum oxide (La$_2$O$_3$), and erbium oxide (Er$_2$O$_3$). Preferably, the stabilizer is yttria.

The stabilizer may be used alone, or two or more thereof may be used in combination.

The stabilizer content in a zirconia pre-sintered body of the present invention and in a sintered body thereof can be measured using a technique, for example, such as inductively coupled plasma (ICP) emission spectral analysis, or x-ray fluorescence analysis.

The stabilizer content in a zirconia pre-sintered body of the present invention and in a sintered body thereof is preferably 0.1 to 18 mol%, more preferably 1 to 15 mol%, even more preferably 1.5 to 10 mol% relative to the total mole of zirconia and stabilizer.

In certain preferred embodiments, it is preferable in the zirconia pre-sintered body and a sintered body thereof that the stabilizer be yttria, and, in view of translucency and strength, the yttria content fall within a range of preferably 2.0 to 8.0 mol%, more preferably 2.2 to 7.5 mol%, even more preferably 2.5 to 7.0 mol%, particularly preferably 2.8 to 6.5 mol% in all of the layers in the layered structure, relative to the total mole of zirconia and stabilizer.

A zirconia pre-sintered body of the present invention comprises a coloring component.

The coloring component is not particularly limited, as long as it imparts a color to the zirconia sintered body. Examples of the coloring component include a pigment, a composite pigment, and a fluorescent agent.

Examples of the pigment include an oxide of at least one element selected from the group consisting of Ti, V, Cr, Mn, Fe, Co, Ni, Zn, Y, Zr, Sn, Sb, Bi, Ce, Pr, Sm, Eu, Gd, Tb, and Er (excluding Y$_2$O$_3$, CeO$_2$, and ZrO$_2$). Preferably, the pigment comprises an oxide of at least one element selected from the group consisting of Ti, V, Cr, Mn, Fe, Co, Ni, Pr, Tb, and Er.

Examples of the composite pigment include (Zr,V)O$_2$, Fe(Fe,Cr)$_2$O$_4$, (Ni,Co,Fe)(Fe,Cr)$_2$O$_4$·ZrSiO$_4$, and (Co,Zn)Al$_2$O$_4$. Preferably, the composite pigment comprises (Zr,V)O$_2$.

Examples of the fluorescent agent include Y$_2$SiO$_5$:Ce, Y$_2$SiO$_5$:Tb, (Y,Gd,Eu)BO$_3$, Y$_2$O$_3$:Eu, YAG:Ce, ZnGa$_2$O$_4$:Zn, and BaMgAl$_{10}$O$_{17}$:Eu.

A zirconia pre-sintered body of the present invention comprises at least two layers that differ from one another in the content of the stabilizer relative to the total mole of zirconia and stabilizer.

The number of layers having different stabilizer contents is not particularly limited, as long as it is 2 or more. The number of layers having different stabilizer contents may be 3 or 4, or may be 5 or more.

In the present specification, "having different stabilizer contents" (for example, yttria contents) means that the difference in stabilizer content is 0.1 mol% or more. The difference in stabilizer content is preferably 0.3 mol% or more, more preferably 0.5 mol% or more.

The difference in stabilizer content is preferably 3.0 mol% or less, more preferably 2.5 mol% or less, even more preferably 2.0 mol% or less, particularly preferably 1.5 mol% or less, most preferably 1.0 mol% or less.

A zirconia pre-sintered body of the present invention also comprises at least two layers having substantially the same stabilizer content relative to the total mole of zirconia and stabilizer.

The number of layers having substantially the same stabilizer content is not particularly limited, as long as it is 2 or more. The number of layers having substantially the same stabilizer content may be 3 or 4, or may be 5 or more.

The layers having substantially the same stabilizer content all contain a coloring component, and differ from one another in the composition of the coloring component.

By comprising layers having different stabilizer contents and layers having substantially the same stabilizer content, and with the layers having substantially the same stabilizer content all containing a coloring component and differing from one another in the composition of the coloring component, a zirconia pre-sintered body of the present invention can achieve a gradual transition of translucency from the cervical to incisal region while reproducing translucency and shade change comparable to those seen in the cervical region of natural teeth, enabling replication of gradational translucency and the translucency and shade of the incisal region, in addition to having an appropriate strength and exhibiting excellent aesthetics.

In view of providing superior aesthetics, it is preferable that the layers having substantially the same stabilizer content and differing in the composition of the coloring component be adjacent to one another.

In the present specification, "having substantially the same stabilizer content" (for example, yttria content) means that the difference in stabilizer content is less than 0.1%. The difference in stabilizer content is preferably less than 0.05%, more preferably less than 0.03%.

As used herein, "differing in the composition of a coloring component" also applies when the layers differ only in the type of coloring component, or when the layers differ only in the content of coloring component. For example, the type and the content of the coloring component contained in each layer can be varied so that the color difference $\Delta E_2^*$ between the shade of a first layer and the shade of a second layer falls within the desired range in two of the layers having substantially the same stabilizer content in a sintered body fabricated by firing the zirconia pre-sintered body at an adequate sintering temperature for 120 minutes, as will be described later.

The coloring component may be contained in at least one of the layers of the zirconia pre-sintered body.

For example, in the preferred embodiment (X-1) below, only one of the second layer and the third layer may contain a coloring component.

With a zirconia pre-sintered body comprising layers having different stabilizer contents and layers having the same stabilizer content and differing in the composition of coloring component, it is possible to appropriately set the translucency, shade, and strength required in different portions (layers) of the same material in a zirconia sintered body made from such a zirconia pre-sintered body. The thickness of each layer is not particularly limited, and may be about 0.5 mm to 3 cm.

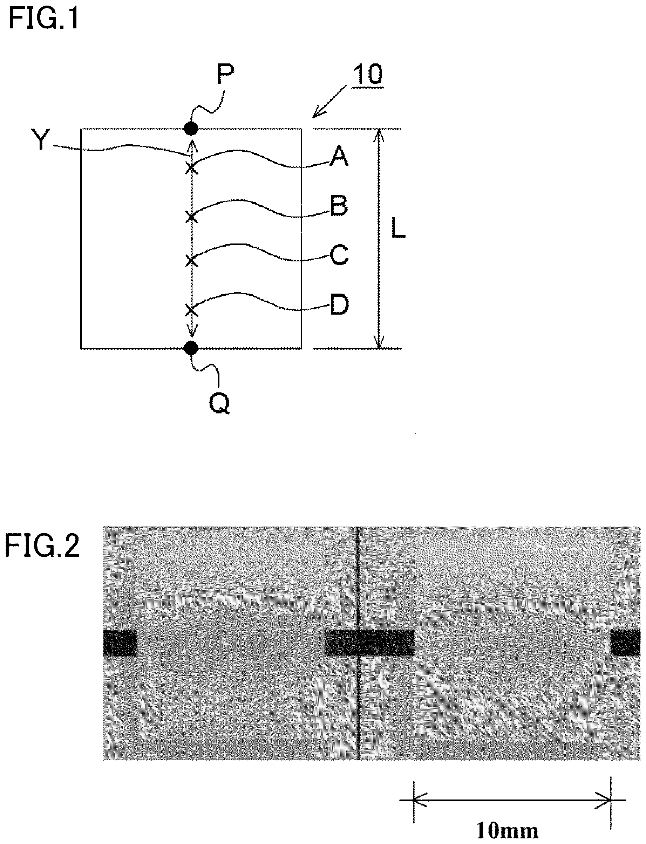

In a zirconia pre-sintered body of the present invention, the thickness of the layer including one end P (the layer corresponding to the incisal region) of the zirconia pre-sintered body 10 of FIG. 1 is not particularly limited. However, in view of ensuring sufficient translucency and shades in the incisal region, the thickness of the layer including one end P ranges preferably from 10 to 45%, more preferably from 15 to 40%, even more preferably from 20 to 35% of the total thickness of the layered structure.

In a zirconia pre-sintered body of the present invention, the thickness of the layer including neither one end P nor the other end Q (the layer corresponding to the middle portion) of the zirconia pre-sintered body 10 of FIG. 1 is not particularly limited. However, in view of facilitating gradual changes in translucency between the incisal region and the cervical region, the thickness of the layer including neither one end P nor the other end Q ranges preferably from 10 to 80%, more preferably from 20 to 70%, even more preferably from 25 to 60% of the total thickness of the layered structure.

In a zirconia pre-sintered body of the present invention, the thickness of the layer including the other end Q (the layer corresponding to the cervical region) of the zirconia pre-sintered body 10 of FIG. 1 is not particularly limited. However, in view of ensuring sufficient strength and shades required for the cervical region, the thickness of the layer including the other end Q ranges preferably from 10 to 45%, more preferably from 15 to 40%, even more preferably from 20 to 35% of the total thickness of the layered structure.

In a zirconia pre-sintered body of the present invention, there may be some overlap between layers having different stabilizer contents and layers having substantially the same stabilizer content. The following describes specific examples.

A certain preferred embodiment (X-1) is, for example, a zirconia pre-sintered body that comprises a layered structure of three layers containing zirconia and a stabilizer capable of preventing a phase transformation of zirconia, and in which a first layer and a second layer have different stabilizer contents, and the second layer and a third layer have substantially the same stabilizer content.

In the embodiment (X-1), the first layer is, for example, the layer including one end P of the zirconia pre-sintered body 10 of FIG. 1, the second layer is the layer adjacent the layer including one end P, and the third layer is the layer adjacent the second layer, for example, the layer including the other end Q of the zirconia pre-sintered body 10 of FIG. 1.

Another preferred embodiment (X-2) is, for example, a zirconia pre-sintered body that comprises a layered structure of four layers containing zirconia and a stabilizer capable of preventing a phase transformation of zirconia, and in which a first layer, a second layer, and a third layer have different stabilizer contents, and the third layer and a fourth layer have substantially the same stabilizer content.

In the embodiment (X-2), the first layer is, for example, the layer including one end P of the zirconia pre-sintered body 10 of FIG. 1, the second layer is the layer adjacent the layer including one end P, the third layer is the layer adjacent the second layer, and the fourth layer is the layer adjacent the third layer, for example, the layer including the other end Q of the zirconia pre-sintered body 10 of FIG. 1.

Another preferred embodiment (X-3) is, for example, a zirconia pre-sintered body that comprises a layered structure of four layers containing zirconia and a stabilizer capable of preventing a phase transformation of zirconia, and in which a first layer and a second layer have different stabilizer contents, and the second layer, a third layer, and a fourth layer have substantially the same stabilizer content.

In the embodiment (X-3), the first layer is, for example, the layer including one end P of the zirconia pre-sintered body 10 of FIG. 1, the second layer is the layer adjacent the layer including one end P, the third layer is the layer adjacent the second layer, and the fourth layer is the layer adjacent the third layer, for example, the layer including the other end Q of the zirconia pre-sintered body 10 of FIG. 1.

In the layered structure of a zirconia pre-sintered body of the present invention, it is preferable that the layer having the highest stabilizer content be a layer that is present at an end face. For example, the layer having the highest stabilizer content may be the layer including one end P on a straight line extending along a first direction Y from one end P to the other end Q of the zirconia pre-sintered body 10 of FIG. 1.

In view of obtaining a suitable shade for dental use, a certain preferred embodiment may be, for example, a zirconia pre-sintered body comprising a layered structure that includes only one layer having the highest stabilizer content.

In a zirconia pre-sintered body of the present invention, the layer having the highest stabilizer content may contain a coloring component. When the layer having the highest stabilizer content contains a coloring component, it is preferable that the composition of the coloring component contained in this layer differ from the compositions of the coloring components contained in the other layers.

An example of such a zirconia pre-sintered body in which the composition of the coloring component contained in the layer having the highest stabilizer content differs from the compositions of the coloring components contained in the other layers is a zirconia pre-sintered body in which the layer having the highest stabilizer content has a different composition of coloring component as a result of changes made by, for example, reducing the amount of coloring component or changing the type of coloring component from the amount or type of the coloring component contained in layers having substantially the same stabilizer content so that the layer having the highest stabilizer content corresponds to a layer including the incisal region.

In another certain embodiment, the stabilizer content in the layer having the highest stabilizer content may be the yttria content of the layer including one end P of the zirconia pre-sintered body 10 described below, when the stabilizer is yttria.

In yet another certain embodiment, the stabilizer content in layers having substantially the same stabilizer content may be the yttria content of the layer including the other end Q of the zirconia pre-sintered body 10 described below, when the stabilizer is yttria.

In view of achieving the shade and strength suited for dental use, it is preferable in a zirconia pre-sintered body of the present invention that the stabilizer (preferably, yttria) content relative to the total mole of zirconia and stabilizer, overall, show an unchanging pattern of increase or decrease from one end P to the other end Q of the zirconia pre-sintered body on a straight line extending along a first direction Y from one end P to the other end Q of the zirconia pre-sintered body, even with a plurality of layers having substantially the same stabilizer (preferably, yttria) content. In other words, it is preferable that the stabilizer (preferably, yttria) content monotonously increase or decrease.

It is also preferable in view of obtaining suitable translucency that the stabilizer (preferably, yttria) content, overall, show an unchanging pattern of increase or decrease on a straight line extending along a first direction Y from one end P to the other end Q of the zirconia pre-sintered body, even with a plurality of layers having substantially the same stabilizer (preferably, yttria) content.

This is described below with reference to FIG. 1 showing a schematic view of a zirconia pre-sintered body.

It is preferable that the pattern of increase or decrease of stabilizer content do not change in the opposite direction on a straight line extending along a first direction Y from one end P to the other end Q of the zirconia pre-sintered body 10 shown in FIG. 1. Specifically, when the stabilizer content is in a pattern of decrease on a straight line from one end P to the other end Q, it is preferable that there exist no interval in which the stabilizer content essentially increases, even with a plurality of layers having the same stabilizer (preferably, yttria) content.

The stabilizer is preferably yttria in view of the strength and translucency of a zirconia sintered body made from a zirconia pre-sintered body of the present invention.

The following describes an embodiment in which the stabilizer is yttria.

The yttria content in the layer including one end P of the zirconia pre-sintered body 10 of FIG. 1 is preferably 3.5 mol% or more, more preferably 3.7 mol% or more, even more preferably 3.8 mol% or more, particularly preferably 4.0 mol% or more, and is preferably 6.5 mol% or less, more preferably 6.0 mol% or less, even more preferably 5.8 mol% or less, particularly preferably 5.5 mol% or less, relative to the total mole of zirconia and yttria.

When the layer including one end P has a yttria content of 3.5 mol% or more and 6.5 mol% or less, the zirconia sintered body can have an increased translucency, appropriate for the incisal region of dental prostheses.

The yttria content of the layer including the other end Q of the zirconia pre-sintered body 10 is preferably 2.5 mol% or more, more preferably 3.0 mol% or more, even more preferably 3.3 mol% or more, particularly preferably 3.5 mol% or more, and is preferably less than 4.5 mol%, more preferably 4.2 mol% or less, even more preferably 4.1 mol% or less, particularly preferably 4.0 mol% or less, relative to the total mole of zirconia and yttria.

When the layer including the other end Q has a yttria content of 2.5 mol% or more and less than 4.5 mol%, the zirconia sintered body can have an increased strength, appropriate for the cervical region of dental prostheses.

With a yttria content of 2.5 mol% or more and less than 4.5 mol%, the translucency does not overly increase, and the zirconia sintered body can show a translucency appropriate for the cervical region of dental prostheses.

Between the layer including one end P and the layer including the other end Q, a zirconia pre-sintered body of the present invention comprises at least one layer as an intermediate layer having a different yttria content from the yttria content of either of the layer including one end P and the layer including the other end Q. In this way, the translucency gradually makes a transition from the cervical region to the incisal region, providing translucency comparable to that of natural teeth.

In a zirconia pre-sintered body of the present invention, it is more preferable in layers having different yttria contents that the yttria content relative to the total mole of zirconia and yttria show an unchanging pattern of increase or decrease from one end to the other end of the zirconia pre-sintered body on a straight line extending along a first direction from one end to the other end of the zirconia pre-sintered body, and that the yttria content of each layer fall within predetermined ranges.

In the zirconia pre-sintered body 10, the yttria content relative to the total mole of zirconia and yttria have a difference of preferably 3.0 mol% or less, more preferably 2.5 mol% or less, even more preferably 2.0 mol% or less between the layer including one end P of the zirconia pre-sintered body 10 and the layer including the other end Q of the zirconia pre-sintered body 10.

The difference of yttria content is preferably 0.3 mol% or more, more preferably 0.5 mol% or more, even more preferably 1.0 mol% or more between the layer including one end P and the layer including the other end Q.

With a yttria content difference of 3.0 mol% or less between the layer including one end P of the zirconia pre-sintered body 10 and the layer including the other end Q of the zirconia pre-sintered body 10, a dental prosthesis made from the zirconia pre-sintered body 10 does not show an overly large translucency difference between the incisal region and the cervical region, and can exhibit a translucency appropriate as a dental prosthesis.

With a yttria content difference of 3.0 mol% or less, the difference between the firing shrinkage rate of the layer including one end P and the firing shrinkage rate of the layer including the other end Q can be confined within 0.3%, and cracking and deformation can be prevented in making a dental prosthesis from zirconia pre-sintered body 10.

In certain embodiments of a zirconia pre-sintered body of the present invention, at least one layer having a different yttria content from the yttria content of either of the layer including one end P and the layer including the other end Q is provided as an intermediate layer between the layer including one end P and the layer including the other end Q.

The difference of yttria content between the intermediate layer and the layer having a different yttria content from the intermediate layer (for example, the layer including one end P, or the layer including the other end Q) is preferably 2.0 mol% or less, more preferably 1.5 mol% or less, even more preferably 1.0 mol% or less.

The yttria content difference is preferably 0.1 mol% or more, more preferably 0.3 mol% or more, even more preferably 0.5 mol% or more.

A certain preferred embodiment is, for example, a zirconia pre-sintered body in which the yttria content difference is more than 0.5 mol% between the intermediate layer and the layer differing in yttria content from the intermediate layer (for example, the layer including one end P, or the layer including the other end Q).

In connection with the above descriptions based on the schematic view of FIG. 1, it is preferable in the present invention that "one end" refer to a point at the incisal end, and "other end" refer to a point at the root (cervical) end, when, for example, the zirconia pre-sintered body and a sintered body thereof have a shape of a crown. The point may be a point on the end face, or a point on a cross section.

When the zirconia pre-sintered body has a disc shape or a hexahedral shape such as a cuboid, it is preferable that "one end" refer to a point on the top face, and "other end" refer to a point on the bottom face (base). The point may be a point on the end face, or a point on a cross section.

In the present invention, "first direction from one end to the other end" means a direction in which the yttria content changes. For example, "first direction" is preferably the direction of lamination of a powder in the production method described below. For example, when the zirconia pre-sintered body has a shape of a crown, "first direction" is preferably a direction connecting the incisal and cervical ends.

A certain embodiment is, for example, a zirconia pre-sintered body that, in addition to any of the configurations of the zirconia pre-sintered bodies described above, shows a changing pattern of increase or decrease in post-sintering L* value according to L*a*b* color system from a first point toward a second point on a straight line connecting the first point and the second point when the first point falling within an interval from one end of the zirconia pre-sintered body to 25% of the entire length on a straight line extending along a first direction from said one end to the other end of the zirconia pre-sintered body has post-sintering (L*, a*, b*) of (L1, a1, b1) according to L*a*b* color system, and the second point falling within an interval from said other end to 25% of the entire length on a straight line extending along the first direction has post-sintering (L*, a*, b*) of (L2, a2, b2) according to L*a*b* color system.

Through the interaction with the stabilizer content in each layer of the layered structure, such zirconia pre-sintered bodies can achieve an even greater balance between the translucency and shade required for the incisal region and the strength and shade needed for the cervical region. This enables the creation of a zirconia pre-sintered body that can be used for a broad range of dental applications.

In view of achieving an even greater balance between the translucency and shade required for the incisal region and the strength and shade needed for the cervical region through the interaction with the stabilizer content in each layer of the layered structure, it is preferable that the zirconia pre-sintered body with a changing pattern of increase or decrease in L* value show an unchanging pattern of increase or decrease in post-sintering a* and b* according to L*a*b* color system from the first point toward the second point.

In view of achieving even greater reproduction of the complex shades and translucency of natural teeth through the interaction with the stabilizer content in each layer of the layered structure, it is preferable in the zirconia pre-sintered body with a changing pattern of increase or decrease in L* value that L1 < L3 > L2 when a third point falling between the first point and the second point on a straight line connecting the first point and the second point has post-sintering (L*, a*, b*) of (L3, a3, b3) according to L*a*b* color system.

In the zirconia pre-sintered body with a changing pattern of increase or decrease in L* value, the difference between L1 and L2 is preferably 10.0 or less, more preferably 8.0 or less, even more preferably 5.0 or less.

In the zirconia pre-sintered body with a changing pattern of increase or decrease in L* value, L1 is preferably 65.0 or more and 93.0 or less, more preferably 67.0 or more and 91.0 or less, even more preferably 70.0 or more and 90.0 or less.

In view of exhibiting the complex shades and translucency of natural teeth with the L* value, it is preferable in the zirconia pre-sintered body with a changing pattern of increase or decrease in L* value that a1 be -3.0 or more and 4.5 or less, more preferably -2.7 or more and 4.0 or less, even more preferably -2.5 or more and 3.7 or less.

In view of exhibiting the complex shades and translucency of natural teeth with the L* value, it is preferable in the zirconia pre-sintered body with a changing pattern of increase or decrease in L* value that b1 be 0.0 or more and 24.0 or less, more preferably 0.3 or more and 23.0 or less, even more preferably 0.5 or more and 22.0 or less.

In view of exhibiting the complex shades and translucency of natural teeth with the L* value, it is preferable in the zirconia pre-sintered body with a changing pattern of increase or decrease in L* value that L2 be 60.0 or more and 92.0 or less, more preferably 63.0 or more and 90.0 or less, even more preferably 65.0 or more and 88.0 or less.

In view of exhibiting the complex shades and translucency of natural teeth with the L* value, it is preferable in the zirconia pre-sintered body with a changing pattern of increase or decrease in L* value that a2 be -2.0 or more and 7.0 or less, more preferably -1.5 or more and 6.5 or less, even more preferably -1.2 or more and 6.0 or less.

In view of exhibiting the complex shades and translucency of natural teeth with the L* value, it is preferable in the zirconia pre-sintered body with a changing pattern of increase or decrease in L* value that b2 be 4.0 or more and 28.0 or less, more preferably 5.5 or more and 26.0 or less, even more preferably 7.0 or more and 24.0 or less.

It is preferable in the zirconia pre-sintered body with a changing pattern of increase or decrease in L* value that a1 < a2, and b1 < b2.

It is preferable in the zirconia pre-sintered body with a changing pattern of increase or decrease in L* value that L3 be 66.0 or more and 95.0 or less, more preferably 69.0 or more and 94.0 or less, even more preferably 72.0 or more and 93.0 or less.

In view of exhibiting the complex shades and translucency of natural teeth with the L* value, it is preferable in the zirconia pre-sintered body with a changing pattern of increase or decrease in L* value that a3 be -2.5 or more and 6.0 or less, more preferably -2.0 or more and 5.7 or less, even more preferably -1.5 or more and 5.5 or less.

In view of exhibiting the complex shades and translucency of natural teeth with the L* value, it is preferable in the zirconia pre-sintered body with a changing pattern of increase or decrease in L* value that b3 be 1.5 or more and 26.0 or less, more preferably 2.0 or more and 25.0 or less, even more preferably 2.5 or more and 23.0 or less.

In view of exhibiting the complex shades and translucency of natural teeth with the L* value, it is preferable in the zirconia pre-sintered body with a changing pattern of increase or decrease in L* value that a1 < a3 < a2, and b1 < b3 < b2.

In view of exhibiting the complex shades and translucency of natural teeth, it is preferable that L4 > L2 when a fourth point falling between the third point and the second point on a straight line connecting the first point and the second point has post-sintering (L*, a*, b*) of (L4, a4, b4) according to L*a*b* color system. L3 and L4 can be varied according to the desired shade and translucency, and may be L3 < L4, or L3 > L4.

In view of exhibiting the complex shades and translucency of natural teeth with the L* value, it is preferable that a4 < a2, and b4 < b2 when the fourth point has post-sintering (L*, a*, b*) of (L4, a4, b4) according to L*a*b* color system.

A zirconia pre-sintered body of the present invention has a flexural strength of preferably 15 MPa or more, in order to provide strength that allows for mechanical working. For easier mechanical working, the zirconia pre-sintered body has a flexural strength of preferably 70 MPa or less, more preferably 60 MPa or less.

The flexural strength can be measured in compliance with ISO 6872:2015. For measurement, a specimen measuring 5 mm × 10 mm × 50 mm is used under the same conditions except for size. For surface finishing, the specimen surfaces, including the chamfered surface (45° chamfers at the corners of specimen), are finished longitudinally with #600 sandpaper. The specimen is disposed in such an orientation that its widest face is perpendicular to the vertical direction (loading direction). In the flexure test, measurements are made at a span of 30 mm with a crosshead speed of 0.5 mm/min.

A zirconia pre-sintered body of the present invention may comprise additives other than the zirconia, stabilizer, and coloring component, provided that the present invention can exhibit its effects.

The layer having the highest stabilizer content may or may not contain a coloring component. Examples of the additives include alumina ($Al_2O_3$), titanium oxide ($TiO_2$), and silica ($SiO_2$).

A zirconia pre-sintered body of the present invention can be made by a process in which a zirconia molded body formed of a raw material powder containing zirconia, a stabilizer, and a coloring component is fired (or pre-sintered) at a temperature that does not sinter the zirconia particles (pre-sintering step).

The stabilizer and coloring component, whether in the form of a raw material powder or pre-sintered body, may or may not be dissolved in zirconia as a solid solution.

The zirconia molded body is not particularly limited, and can be produced by a known method (for example, press forming), using a raw material powder containing zirconia particles and a stabilizer.

In order to ensure block formation, the firing temperature (pre-sintering temperature) in the pre-sintering step is, for example, preferably 800°C or more, more preferably 900°C or more, even more preferably 950°C or more. For increased processability, the pre-sintering temperature is, for example, preferably 1,200°C or less, more preferably 1,150°C or less, even more preferably 1,100°C or less. For example, the pre-sintering temperature in a method of production of a zirconia pre-sintered body of the present invention is preferably 800°C to 1,200°C.

A zirconia pre-sintered body of the present invention may be a molded body having a predetermined shape. For example, the zirconia pre-sintered body may have a shape of a disc (discotic), a cuboid, or a dental product (for example, a shape of a crown). The pre-sintered body also encompasses dental products (for example, a crown-shaped dental prosthesis) obtained after working of a pre-sintered zirconia disc with a CAD/CAM (Computer-Aided Design/Computer-Aided Manufacturing) system.

In a zirconia pre-sintered body of the present invention, the crystal system of zirconia is not limited, and the predominant crystal system may be monoclinic, tetragonal, or cubic.

A certain embodiment is, for example, a zirconia pre-sintered body that is predominantly monoclinic in crystal system.

In this specification, "being predominantly monoclinic in crystal system" means that the content $f_m$ of the monoclinic crystal system of zirconia calculated from the formula (1) below is 50% or more relative to the total amount of all the crystal systems (monoclinic, tetragonal, and cubic) in zirconia.

The content $f_m$ of the monoclinic crystal system can be calculated by the following formula (1) using peaks in an X-ray diffraction (XRD) pattern by CuK$\alpha$ radiation.

$$f_m = I_m/(I_m + I_t + I_c) \times 100 \qquad (1)$$

where $f_m$ represents the content (%) of the monoclinic crystal system in the zirconia raw material, $I_m$ represents the area intensity of the main peak of the monoclinic crystal system near $2\theta = 28°$ in XRD measurement, It represents the area intensity of the main peak of the tetragonal crystal system near $2\theta = 30°$ in XRD measurement, and $I_c$ represents the area intensity of the main peak of the cubic crystal system near $2\theta = 30°$ in XRD measurement. Because the main peak positions of the tetragonal and cubic crystal systems are in proximity, information about the positions of sub-peaks is also taken into account to determine which crystal system is present. When it is determined that both tetragonal and cubic crystal systems are present, the area intensity is calculated for the main peaks of these crystal systems through peak separation.

In certain embodiments, the peaks for tetragonal and cubic crystal systems may be essentially undetectable in a zirconia pre-sintered body of the present invention. That is, the content $f_m$ of the monoclinic crystal system may be 100% in the zirconia pre-sintered body.

In certain embodiments, the content $f_m$ of the monoclinic crystal system in zirconia calculated from the formula (1) above is preferably 55% or more relative to the total amount of the monoclinic, tetragonal, and cubic crystal systems in a zirconia pre-sintered body of the present invention. In view of reducing the firing time and achieving suitable translucency and excellent strength for dental applications, the content $f_m$ is more preferably 60% or more, even more preferably 70% or more, yet more preferably 75% or more, particularly preferably 80% or more, still more preferably 85% or more, most preferably 90% or more.

Another embodiment is, for example, a zirconia pre-sintered body that is predominantly tetragonal and/or cubic in crystal system.

Here, "being predominantly tetragonal and/or cubic in crystal system" means that the content ft of the tetragonal crystal system and/or the content $f_c$ of the cubic crystal system in zirconia calculated from the formulae (2) and/or (3) below are 50% or more relative to the total amount of all the crystal systems (monoclinic, tetragonal, and cubic) in zirconia.

The content ft of the tetragonal crystal system can be calculated by the following formula (2) using peaks in an X-ray diffraction (XRD) pattern by CuK$\alpha$ radiation.

$$f_t = I_t/(I_m + I_t + I_c) \times 100 \qquad (2)$$

where ft represents the content (%) of the tetragonal crystal system in the zirconia raw material, and $I_m$, It, and $I_c$ in XRD measurement are as defined in formula (1).

The content $f_c$ of the cubic crystal system can be calculated by the following formula (3) using peaks in an X-ray diffraction (XRD) pattern by CuKα radiation.

$$f_c = I_c/(I_m + I_t + I_c) \times 100 \qquad (3)$$

where $f_c$ represents the content (%) of the cubic crystal system in the zirconia raw material, and $I_m$, $I_t$, and $I_c$ in XRD measurement are as defined in formula (1).

In certain embodiments, the content ft of the tetragonal crystal system in zirconia calculated from the formula (2) above is preferably 55% or more relative to the total amount of the monoclinic, tetragonal, and cubic crystal systems in a zirconia pre-sintered body of the present invention. In view of achieving suitable translucency and excellent strength for dental applications, the content ft is more preferably 60% or more, even more preferably 70% or more, yet more preferably 75% or more, particularly preferably 80% or more, still more preferably 85% or more, most preferably 90% or more.

In another certain embodiment, the content ft of the tetragonal crystal system and the content $f_c$ of the cubic crystal system in zirconia calculated from the formulae (2) and (3) above are preferably 55% or more relative to the total amount of the monoclinic, tetragonal, and cubic crystal systems in a zirconia pre-sintered body of the present invention. In view of achieving suitable translucency and excellent strength for dental applications, the content ft and content $f_c$ are more preferably 60% or more, even more preferably 70% or more, yet more preferably 75% or more, particularly preferably 80% or more, still more preferably 85% or more, most preferably 90% or more.

In yet another certain embodiment, the content $f_c$ of the cubic crystal system in zirconia calculated from the formula (3) above is preferably 55% or more relative to the total amount of the monoclinic, tetragonal, and cubic crystal systems in a zirconia pre-sintered body of the present invention. In view of achieving suitable translucency for dental applications, the content $f_c$ is more preferably 60% or more, even more preferably 70% or more, yet more preferably 75% or more, particularly preferably 80% or more, still more preferably 85% or more, most preferably 90% or more.

In a zirconia pre-sintered body of the present invention, the layered structure includes at least two layers having substantially the same stabilizer content, as described above. In other words, the layered structure includes at least a first and a second layer having substantially the same stabilizer content.

In certain embodiments, the zirconia pre-sintered body has a color difference $\Delta E_2^*$ of preferably 0.3 or more, more preferably 0.4 or more, even more preferably 0.5 or more in the formula (4) below representing the difference in shade between a first layer and a second layer when the shade $(L_1^*, a_1^*, b_1^*)$ of the first layer and the shade $(L_2^*, a_2^*, b_2^*)$ of the second layer are compared for two of the layers having substantially the same stabilizer content in a sintered body fabricated by firing the zirconia pre-sintered body at an adequate sintering temperature for 120 minutes, in order for the sintered body to reproduce the shade change seen in the cervical region of natural teeth.

The color difference $\Delta E_2^*$ is preferably 6.0 or less, more preferably 5.0 or less, even more preferably 4.5 or less, particularly preferably 4.0 or less.

$$\Delta E_2^* = \{(L_2^* - L_1^*)^2 + (a_2^* - a_1^*)^2 + (b_2^* - b_1^*)^2\}^{1/2} \qquad (4)$$

In the formula, $(L_1^*, a_1^*, b_1^*)$ represents the shade of the first layer in the layers having substantially the same stabilizer content, and $(L_2^*, a_2^*, b_2^*)$ represents the shade of the second layer in the layers having substantially the same stabilizer content.

In another certain embodiment, it is preferable that the color difference $\Delta E_2^*$ between adjacent layers of all the layers having substantially the same stabilizer content in the layered structure of a zirconia pre-sintered body of the present invention fall within the foregoing ranges, in order for the sintered body to reproduce the shade change seen in the cervical region of natural teeth.

The following describes a zirconia sintered body of the present invention.

In this specification, a zirconia sintered body is, for example, a body of zirconia particles (powder) that has been sintered. Particularly, a zirconia sintered body of the present invention refers to a zirconia sintered body made from a zirconia pre-sintered body of the present invention. The zirconia sintered body has a relative density of preferably 99.5% or more. The relative density can be calculated as a ratio of the actual density, measured by the Archimedes method, with respect to the theoretical density.

A zirconia sintered body of the present invention encompasses not only sintered bodies after sintering of a molded zirconia particles under ordinary pressure or no applied pressure, but sintered bodies compacted by a high-temperature pressing process such as HIP (Hot Isostatic Pressing).

A zirconia sintered body of the present invention has the same zirconia and stabilizer content as the pre-sintered body to be made into a sintered body.

The following describes a method of production of a zirconia sintered body of the present invention.

A zirconia sintered body of the present invention can be made by firing a zirconia pre-sintered body at a temperature (sinterable temperature) that sinters the zirconia particles (sintering step). The sinterable temperature (for example, highest sintering temperature) in the sintering step is, for example, preferably 1,400°C or more, more preferably 1,450°C or more. The sinterable temperature is, for example, preferably 1,650°C or less, more preferably 1,600°C or less.

The rate of temperature increase and the rate of temperature decrease are preferably 300°C/min or less. That is, it is preferable in a method of production of a zirconia sintered body of the present invention that the zirconia pre-sintered body be fired at a highest sintering temperature of 1,400°C to 1,650°C. The highest sintering temperature may be the adequate sintering temperature of the zirconia pre-sintered body.

In the sintering step, the retention time at the sinterable temperature (for example, highest sintering temperature) may be 120 minutes. However, the retention time is not particularly limited, and may be, for example, 120 minutes or less, 90 minutes or less, 75 minutes or less, 60 minutes or less, 45 minutes or less, 30 minutes or less, or 15 minutes or less. The retention time is preferably 1 minute or more, more preferably 5 minutes or more, even more preferably 10 minutes or more.

The rate of temperature increase and the rate of temperature decrease in the sintering step are not particularly limited. The rate of temperature increase to the highest sintering temperature may be, for example, 5°C/min or more, 10°C/min or more, 50°C/min or more, 100°C/min or more, 120°C/min or more, 150°C/min or more, or 200°C/min or more. Preferably, the rate of temperature decrease from the highest sintering temperature is set to a rate that does not cause cracks or other defects in the sintered body. For example, the sintered body may be allowed to cool at room temperature after being heated. Here, the highest sintering temperature means the highest temperature reached in the sintering step.

A zirconia sintered body after firing of a zirconia pre-sintered body of the present invention can be suitably used as a dental product. Examples of dental products include copings, frameworks, crowns, crown bridges, long-span bridges, abutments, implants, implant screws, implant fixtures, implant bridges, implant bars, brackets, denture bases, inlays, onlays, orthodontic wires, and laminate veneers.

The method of producing these dental products can be appropriately selected depending on use. For example, a dental product can be obtained by sintering a zirconia pre-sintered body of the present invention after milling. Preferably, a CAD/CAM system is used for milling.

The present invention encompasses embodiments combining the foregoing features, provided that the present invention can exhibit its effects with such combinations made in various forms within the technical idea of the present invention.

EXAMPLES

The following describes the present invention in greater detail by way of Examples. It should be noted, however, that the present invention is in no way limited by the following Examples, and various changes may be made by a person with ordinary skill in the art within the technical idea of the present invention.

[Fabrication of Zirconia Pre-Sintered Body]

Zirconia pre-sintered bodies of Examples and Comparative Examples were fabricated using the following procedures.

The raw material powders used to fabricate zirconia pre-sintered bodies of Examples 1 to 3 and Comparative Examples 1 and 2 were prepared as follows.

First, a monoclinic zirconia powder and a yttria powder were used to prepare a mixture in the compositions shown in Table 1. The mixture was added to water to prepare a slurry, and the slurry was pulverized to an average particle diameter of 0.13 μm or less by wet pulverization through mixing using a ball mill. After pulverization, the slurry was dried with a spray dryer, and the resulting powder was fired at 950°C for 2 hours to prepare a powder (primary powder).

The average particle diameter was determined by a laser diffraction scattering method. For the measurement by a laser diffraction scattering method, a laser diffraction particle size distribution analyzer (SALD-2300, manufactured by Shimadzu Corporation) was used with a 0.2% sodium hexametaphosphate aqueous solution used as a dispersion medium.

A coloring component was added to the primary powder in the compositions shown in Table 1. After adding the coloring component, the powder was added to water to prepare a slurry, and the slurry was pulverized to an average particle diameter of 0.13 $\mu$m or less by wet pulverization through mixing using a ball mill. After adding a binder to the pulverized slurry, the slurry was dried with a spray dryer to prepare a powder (secondary powder). The secondary powder was used as a raw material powder for the production of a zirconia pre-sintered body, as described below.

The following raw material powders were used for the fabrication of zirconia pre-sintered bodies according to Examples 4 and 5 and Comparative Example 3. Zpex® Smile (manufactured by Tosoh Corporation) for layer 1 of Examples 4 and 5, Zpex® 4 (manufactured by Tosoh Corporation) for layers 2 and 3 of Example 4, layer 2 of Example 5, and layers 1 to 3 of Comparative Example 3, and Zpex® (manufactured by Tosoh Corporation) for layers 3 and 4 of Example 5.

The zirconia pre-sintered bodies were produced using the following method.

First, the raw material powders were filled into a 20 mm × 25 mm die (inside dimensions) in the order shown in Table 1, and pressed at a surface pressure of 300 kg/cm$^2$ for 90 seconds with a uniaxial pressing machine (primary pressing). The molded body after primary pressing was then subjected to CIP molding at 1,700 kg/cm$^2$ for 5 minutes to prepare a molded body having a layered structure.

For each layer, 7.5 g of raw material powder was filled in the four-layer structures of Examples 3 and 5, and 10 g of raw material powder was filled in the three-layer structures of Examples 1, 2, and 4 and Comparative Examples 1 to 3. The molded body was fired at 1,000°C for 2 hours to prepare a zirconia pre-sintered body.

Concerning the crystal system of the zirconia pre-sintered body, the predominant crystal system of zirconia was monoclinic in Examples 1 to 3 and Comparative Examples 1 and 2, cubic in layer 1 of Examples 4 and 5 with Zpex® Smile, tetragonal or cubic in layers 2 and 3 of Example 4, layer 2 of Example 5, and layers 1 to 3 of Comparative Example 3 with Zpex® 4, and tetragonal in layers 3 and 4 of Example 5 with Zpex®.

[Definition and Measurement of Adequate Sintering Temperature of Zirconia Pre-Sintered Body]

In the present invention, the adequate sintering temperature of the zirconia pre-sintered body refers to the sintering temperature specified by the manufacturer when commercially available zirconia is used.

In the absence of specific information of sintering temperature, the adequate sintering temperature can be specified as follows.

First, a plurality of zirconia pre-sintered bodies of the same shape was heated to various temperatures at a rate of temperature increase of 10°C/min. After firing the zirconia pre-sintered bodies for 120 minutes at various temperatures, the both sides were polished with #600 abrasive paper to obtain a 0.5 mm-thick specimen of zirconia sintered body.

By visually observing the appearance of the specimen, the adequate sintering temperature of each zirconia pre-sintered body was determined from the transparency of the specimen, using the following criteria.

The zirconia pre-sintered body can be determined as being sufficiently fired when the transparency is high enough to allow the background to be seen through the zirconia pre-sintered body, as in the specimen on the left-hand side of FIG. 2.

On the other hand, firing can be determined as being insufficient when the zirconia pre-sintered body is seen as being low in transparency or being clouded, as in the specimen on the right-hand side of FIG. 2.

In the present invention, the lowest temperature at which the zirconia pre-sintered body can be determined as being sufficiently fired as in the specimen on the left-hand side of FIG. 2 was determined as the adequate sintering temperature of the zirconia pre-sintered body.

In the zirconia pre-sintered body, the adequate sintering temperature in the layer with the highest yttria content is deemed as the adequate sintering temperature of the zirconia pre-sintered body.

The adequate sintering temperature measured for the zirconia pre-sintered body of each Example and Comparative Example in this fashion was 1,500°C for Example 1, and 1,550°C for Examples 2 and 3 and Comparative Examples 1 and 2.

The firing temperature specified by the manufacturer is 1,450°C for Zpex® and Zpex® Smile (manufactured by Tosoh Corporation), and 1,500°C for Zpex® 4 (manufactured by Tosoh Corporation).

In Examples 4 and 5 in which the firing temperature specified by the manufacturer is different between layers, the higher firing temperature was employed so that the adequate sintering temperature is 1,500°C in Examples 4 and 5.

The adequate sintering temperature is 1,500°C in Comparative Example 3 in which Zpex® 4 (manufactured by Tosoh Corporation) was used for all layers.

[Aesthetic Evaluation of Zirconia Sintered Body]

The zirconia pre-sintered body of each Example and Comparative Example was used to fabricate a zirconia sintered body by the method below, and the zirconia sintered body was visually evaluated for aesthetic quality in comparison to the appearance of natural teeth.

A commercially available shade guide providing shades similar in appearance to natural teeth was used for evaluation. Specifically, the VITA Classical shade guide was used (manufactured by VITA under this trade name).

The zirconia pre-sintered body 10 of each Example and Comparative Example fabricated by using the above method was milled into a crown shape, using a CAD/CAM system (KATANA® CAD/CAM system, Kuraray Noritake Dental Inc.). After milling, the zirconia pre-sintered body was fired at the adequate sintering temperature for 120 minutes to fabricate a zirconia sintered body.

The zirconia sintered bodies all had a length of about 8 mm along the direction of lamination. The zirconia sintered bodies were visually evaluated using the following criteria (n = 1). The results are presented in Table 1.

<Evaluation Criteria>

Good: Shows appropriate degrees of variation in translucency and shade gradation in different locations (incisal, middle, and cervical portions) resembling natural teeth, faithfully reproducing the natural teeth aesthetics.

Moderate: The translucency and shades are similar to natural teeth, and there is a gradation in translucency and/or shade, but the variation is monotonous, failing to faithfully reproduce the natural teeth aesthetics.

Poor: Lacks sufficient variation in translucency or shade, failing to reproduce the natural teeth aesthetics.

(Examples 1 to 5 and Comparative Examples 1 to 3)

In Examples 1 to 5, the zirconia sintered bodies all showed a gradation with gradually decreasing translucency and darker shades from a region corresponding to the layer including one end P to a region corresponding to the layer including the other end Q of the zirconia pre-sintered body 10 shown in FIG. 1, and the appearance was comparable to that of natural teeth by reproducing the translucency and the shade change of the cervical region, and the translucency and shade of the incisal region.

In Comparative Example 1 corresponding to Patent Literature 1, the translucency change was insufficient because of the same yttria content across all layers, though the shade had the tendency to be darker from a region corresponding to the layer including one end P to a region corresponding to the layer including the other end Q of the zirconia pre-sintered body 10 shown in FIG. 1. This resulted in the failure to achieve gradational translucency with a gradual transition from the cervical region to the incisal region while reproducing translucency comparable to that seen in the cervical region of natural teeth. In this respect, it was not possible to say that the appearance was comparable to natural teeth.

In Comparative Example 2, it was not possible to achieve the translucency and the shade change seen in the cervical region of natural teeth due to the variation in both yttria content and the composition of the coloring component, and the zirconia sintered body could not be said as being comparable to natural teeth, though the variation in yttria content and pigment content produced a gradation with gradually decreasing translucency and darker shades from a region corresponding to the layer including one end P to a region corresponding to the layer including the other end Q of the zirconia pre-sintered body 10 shown in FIG. 1.

In Comparative Example 3, the translucency change was insufficient because of the same yttria content across all layers, and it was not possible to say that the appearance was comparable to natural teeth, though the shade had the tendency to be darker from a region corresponding to the layer including one end P to a region corresponding to the layer including the other end Q of the zirconia pre-sintered body 10 shown in FIG. 1, as in Comparative Example 1.

Specifically, it can be understood from Comparative Examples 1 and 3 that faithful reproduction of the aesthetic appearance of natural teeth, where the shade and translucency of enamel, dentin, and pulp intricately influence one another, is difficult to achieve with the attempt to bring about variation only with the pigment composition (type and content).

In Comparative Example 2, it was not possible to achieve translucency and shade change in the cervical region, though the variation in yttria content and pigment content produced gradations.

Additionally, the result of Comparative Example 2 indicates that while sintering zirconia pre-sintered bodies with varying yttria content yields incisal portions with excellent translucency and shade, it cannot yield zirconia sintered bodies that also exhibit translucency and shade change in the cervical region. It can be understood from this that it is difficult with such zirconia pre-sintered bodies to faithfully reproduce the appearance of natural teeth, which involves an intricate interplay of shades and translucency in enamel, dentin, and pulp, across the incisal, middle, and cervical portions of the zirconia sintered body obtained after sintering.

In contrast, the zirconia sintered bodies of Examples 1 to 5 exhibited translucency and shade change similar to those seen in the cervical region of natural teeth, and showed a gradual transition of translucency from the cervical region to the incisal region, including the middle portion, reproducing the gradation of translucency with the translucency and shade of the incisal region of natural teeth, without distinct boundaries. With the shade variation and translucency gradation integrally interacting with each other without interfering, the zirconia sintered bodies exhibited an appearance comparable to natural teeth, where each structure influences the others through an intricate interplay.

The zirconia sintered bodies of Examples 1 to 5 also exhibited increasing strength from the incisal region toward the cervical region, with sufficiently high strength in the middle and cervical portions, showing strength appropriate for dental applications.

[Shade Evaluation of Zirconia Sintered Body]

For the measurement of the shade of each layer in the zirconia sintered body of each Example and Comparative Example, a zirconia sintered body was individually prepared for each layer using the method below, and was measured for (L*,a*,b*) of L*a*b* color system (JIS Z 8781-4:2013 Color Measurements - Part 4: CIE 1976 L*a*b* color space).

First, a molded body was prepared with the raw material powder of each layer of Examples and Comparative Examples by pressing the raw material powder in a size pre-adjusted to obtain a zirconia sintered body that becomes 1.2 mm thick after polishing the both surfaces with #600 abrasive paper. The molded body was fired at 1,000°C for 2 hours to prepare a zirconia pre-sintered body. After setting the adequate sintering temperature, the zirconia pre-sintered body was fired for 120 minutes to prepare a zirconia sintered body. The zirconia sintered body was polished with #600 abrasive paper on both sides to obtain a zirconia sintered body having a thickness of 1.2 mm. Subsequently, the shade was measured against a white background using a spectrophotometer CM-3610A (manufactured by Konica Minolta Inc.) with a D65 illuminant in measurement mode SCI, with a measurement area/illumination area of 8 mm/11 mm in diameter.

The color difference $\Delta E_2^*$ between the second and third layers of Examples 1, 2, and 4, and the color difference $\Delta E_2^*$ between the third and fourth layers of Examples 3 and 5 can be calculated using the following formula (4).

$$\Delta E_2^* = \{(L_2^* - L_1^*)^2 + (a_2^* - a_1^*)^2 + (b_2^* - b_1^*)^2\}^{1/2} \qquad (4)$$

In the formula, $(L_1^*, a_1^*, b_1^*)$ represents the shade of a first layer (layer 2 of Examples 1, 2, and 4, and layer 3 of Examples 3 and 5) in the layers having substantially the same stabilizer content, and $(L_2^*, a_2^*, b_2^*)$ represents the shade of a second layer (layer 3 of Examples 1, 2, and 4, and layer 4 of Examples 3 and 5) in the layers having substantially the same stabilizer content.

From the calculated shade values presented in Table 1, Examples 1 to 3 were shown to successfully reproduce the shade variation of the cervical region, imparting shades comparable to natural teeth together with the incisal region as visually confirmed.

[Table 1]

| | Specifications of zirconia pre-sintered body | | | | Adequate sintering temperature | Aesthetics | Shade | | |
|---|---|---|---|---|---|---|---|---|---|
| | Layers | Yttria content | Coloring component | | | | L* | a* | b* |
| | | | NiO | (Zr,V)O$_2$ | | | | | |
| Ex. 1 | Layer 1 | 4 mol% | 0 | 0 | 1500°C | Good | 89.3 | -1.0 | 0.8 |
| | Layer 2 | 3 mol% | 0.003 | 0.006 | | | 89.5 | -0.6 | 5 |
| | Layer 3 | 3 mol% | 0.0035 | 0.008 | | | 88.2 | -0.1 | 8.2 |
| Ex. 2 | Layer 1 | 5 mol% | 0.012 | 0.019 | 1550°C | Good | 82 | -0.5 | 17 |
| | Layer 2 | 4 mol% | 0.015 | 0.025 | | | 83.9 | 0.1 | 18.8 |
| | Layer 3 | 4 mol% | 0.02 | 0.032 | | | 82.5 | 0.8 | 20.5 |
| Ex. 3 | Layer 1 | 6 mol% | 0 | 0 | 1550°C | Good | 86.1 | -1.1 | 0.2 |
| | Layer 2 | 5 mol% | 0.002 | 0.003 | | | 88 | -0.8 | 3.5 |
| | Layer 3 | 4 mol% | 0.003 | 0.005 | | | 88.6 | -0.4 | 6.5 |
| | Layer 4 | 4 mol% | 0.005 | 0.009 | | | 86.3 | -0.1 | 8.9 |

(continued)

| | | Specifications of zirconia pre-sintered body | | | | Adequate sintering temperature | Aesthetics | Shade | | |
|---|---|---|---|---|---|---|---|---|---|---|
| | | Layers | Yttria content | Coloring component | | | | L* | a* | b* |
| | | | | NiO | $(Zr,V)O_2$ | | | | | |
| Ex. 4 | | Layer 1 | 5.3 mol% | 0 | 0 | 1500°C | Good | 89.2 | -1.3 | 0.4 |
| | | Layer 2 | 4 mol% | 0.003 | 0.006 | | | 89.9 | -1.2 | 5.5 |
| | | Layer 3 | 4 mol% | 0.006 | 0.012 | | | 88.9 | -0.8 | 8.8 |
| Ex. 5 | | Layer 1 | 5.3 mol% | 0 | 0 | 1500°C | Good | 89.2 | -1.3 | 0.4 |
| | | Layer 2 | 4 mol% | 0.003 | 0.006 | | | 89.9 | -1.2 | 5.5 |
| | | Layer 3 | 3 mol% | 0.006 | 0.012 | | | 89.5 | -0.8 | 9.1 |
| | | Layer 4 | 3 mol% | 0.01 | 0.02 | | | 87.4 | -0.2 | 15.4 |

[Table 1] Continued

| | | Specifications of zirconia pre-sintered body | | | | Adequate sintering temperature | Aesthetics | Shade | | |
|---|---|---|---|---|---|---|---|---|---|---|
| | | Layers | Yttria content | Coloring component | | | | L* | a* | b* |
| | | | | NiO | $(Zr,V)O_2$ | | | | | |
| Com. Ex. 1 | | Layer 1 | 5 mol% | 0.012 | 0.019 | 1550°C | Poor | 82 | -0.5 | 17 |
| | | Layer 2 | 5 mol% | 0.015 | 0.025 | | | 81.3 | -0.1 | 17.9 |
| | | Layer 3 | 5 mol% | 0.02 | 0.032 | | | 79.3 | 0.8 | 19.8 |
| Com. Ex. 2 | | Layer 1 | 6 mol% | 0.012 | 0.019 | 1550°C | Moderate | 80.5 | -0.4 | 16.2 |
| | | Layer 2 | 5 mol% | 0.015 | 0.025 | | | 81.3 | -0.1 | 17.9 |
| | | Layer 3 | 4 mol% | 0.02 | 0.032 | | | 82.5 | 0.8 | 20.5 |
| Com. Ex. 3 | | Layer 1 | 4 mol% | 0 | 0 | 1500°C | Moderate | 91.1 | -1.3 | 0.8 |
| | | Layer 2 | 4 mol% | 0.003 | 0.006 | | | 89.9 | -1.2 | 5.5 |
| | | Layer 3 | 4 mol% | 0.006 | 0.012 | | | 88.9 | -0.8 | 8.8 |

In the table, the yttria content represents the content of yttria relative to the total mole of zirconia and yttria.

[Measurement of Flexural Strength of Zirconia Sintered Body]

(Examples 1 to 3)

The raw material powders for layer 3 of Example 1, layer 3 of Example 2, and layer 4 of Example 3 were used to prepare zirconia pre-sintered bodies following the method of production of pre-sintered body described above, and the zirconia pre-sintered bodies were fired for 120 minutes at the adequate sintering temperatures of Table 1 to yield zirconia sintered bodies. The zirconia sintered bodies were measured for flexural strength according to ISO6872 using samples measuring 1.2 mm × 4.0 mm × 16.0 mm in size, with the distance between supports (span length) set at 12 mm, and the crosshead speed set to 0.5 mm/min. The flexural strength was 1,310 MPa for layer 3 of Example 1, 1,157 MPa for layer 3 of Example 2, and 1,170 MPa for layer 4 of Example 3, confirming that the zirconia sintered bodies had the strength necessary for the cervical region of dental prostheses.

The numeric ranges given in this specification should be construed such that all numerical values and ranges falling within the ranges specified herein are specifically recited in the specification, even in the absence of specific recitations.

INDUSTRIAL APPLICABILITY

A zirconia pre-sintered body of the present invention and a sintered body thereof can be used for dental products such as prostheses.

Reference Signs List

| 10 | Zirconia pre-sintered body |
| P | One end |
| Q | Other end |
| L | Entire length |
| Y | First direction |

**Claims**

1.  A zirconia pre-sintered body comprising a layered structure of at least three layers containing zirconia and a stabilizer capable of preventing a phase transformation of zirconia,

    the layered structure including at least two layers that differ from one another in the content of the stabilizer relative to the total mole of the zirconia and the stabilizer,
    the layered structure including at least two layers having substantially the same stabilizer content relative to the total mole of the zirconia and the stabilizer,
    the zirconia pre-sintered body containing a coloring component in all of the layers having substantially the same stabilizer content, and the layers having substantially the same stabilizer content differing from one another in the composition of the coloring component.

2.  The zirconia pre-sintered body according to claim 1, wherein the layers having substantially the same stabilizer content are adjacent to one another.

3.  The zirconia pre-sintered body according to claim 1 or 2, wherein the coloring component comprises an oxide of at least one element selected from the group consisting of Ti, V, Cr, Mn, Fe, Co, Ni, Pr, Tb, and Er; or $(Zr,V)O_2$.

4.  The zirconia pre-sintered body according to any one of claims 1 to 3, wherein the layered structure includes only one layer having the highest stabilizer content.

5.  The zirconia pre-sintered body according to any one of claims 1 to 4, wherein the layer having the highest stabilizer content does not contain a coloring component.

6.  The zirconia pre-sintered body according to any one of claims 1 to 4, wherein the layer having the highest stabilizer content contains a coloring component.

7.  The zirconia pre-sintered body according to claim 6, wherein the coloring component contained in the layer having the highest stabilizer content differs in composition from the coloring components of the other layers.

8.  The zirconia pre-sintered body according to any one of claims 1 to 7, wherein, in the layers that differ from one another in the content of the stabilizer, the stabilizer content relative to the total mole of the zirconia and the stabilizer shows an unchanging pattern of increase or decrease from one end to the other end of the zirconia pre-sintered body on a straight line extending along a first direction from one end to the other end of the zirconia pre-sintered body.

9.  The zirconia pre-sintered body according to any one of claims 1 to 8, wherein the stabilizer is yttria.

10. The zirconia pre-sintered body according to claim 9, wherein, in the layers that differ from one another in the content of yttria, the stabilizer content relative to the total mole of the zirconia and the stabilizer shows an unchanging pattern of increase or decrease from one end to the other end of the zirconia pre-sintered body on a straight line extending along a first direction from one end to the other end of the zirconia pre-sintered body, and wherein the yttria content relative to the total mole of the zirconia and the yttria is 3.5 mol% or more and 6.5 mol% or less in the layer including said one end, and is 2.5 mol% or more and less than 4.5 mol% in the layer including said other end.

11. The zirconia pre-sintered body according to claim 10, wherein, in the layers that differ from one another in the content of yttria, the stabilizer content relative to the total mole of the zirconia and the stabilizer shows an unchanging pattern of increase or decrease from one end to the other end of the zirconia pre-sintered body on a straight line extending along a first direction from one end to the other end of the zirconia pre-sintered body, and wherein the difference in yttria content between the layer including said one end and the layer including said other end is 3.0 mol% or less relative to the total mole of the zirconia and the yttria.

12. The zirconia pre-sintered body according to any one of claims 1 to 11, which has a color difference $\Delta E_2^*$ of 0.3 or more and 6.0 or less in the formula (4) below representing the difference in shade between a first layer and a second layer of when the shade $(L_1^*, a_1^*, b_1^*)$ of the first layer and the shade $(L_2^*, a_2^*, b_2^*)$ of the second layer are compared for two of the layers having substantially the same stabilizer content in a sintered body fabricated by firing the zirconia pre-sintered body at an adequate sintering temperature for 120 minutes,

$$\Delta E_2^* = \{(L_2^* - L_1^*)^2 + (a_2^* - a_1^*)^2 + (b_2^* - b_1^*)^2\}^{1/2} \qquad (4)$$

where $(L_1^*, a_1^*, b_1^*)$ represents the shade of the first layer in the layers having substantially the same stabilizer content, and $(L_2^*, a_2^*, b_2^*)$ represents the shade of the second layer in the layers having substantially the same stabilizer content.

13. The zirconia pre-sintered body according to any one of claims 1 to 12, which shows a changing pattern of increase or decrease in post-sintering L* value according to L*a*b* color system from a first point toward a second point on a straight line connecting the first point and the second point when the first point falling within an interval from one end of the zirconia pre-sintered body to 25% of the entire length on a straight line extending along a first direction from said one end to the other end of the zirconia pre-sintered body has post-sintering (L*, a*, b*) of (L1, a1, b1) according to L*a*b* color system, and the second point falling within an interval from said other end to 25% of the entire length on a straight line extending along the first direction has post-sintering (L*, a*, b*) of (L2, a2, b2) according to L*a*b* color system.

14. The zirconia pre-sintered body according to claim 13, wherein L1 < L3 > L2 when a third point falling between the first point and the second point on a straight line connecting the first point and the second point has post-sintering (L*, a*, b*) of (L3, a3, b3) according to L*a*b* color system.

15. A method for producing a zirconia sintered body, comprising sintering a zirconia pre-sintered body of any one of claims 1 to 14 at a highest sintering temperature of 1,400°C to 1,650°C.

16. A method for producing a dental product, comprising sintering a zirconia pre-sintered body of any one of claims 1 to 14 after milling.

17. The method according to claim 16, wherein the milling uses a CAD/CAM system.

## FIG.1

## FIG.2

10mm

<div align="center">

**INTERNATIONAL SEARCH REPORT**

</div>

| | |
|---|---|
| International application No. |
| **PCT/JP2022/048500** |

### A. CLASSIFICATION OF SUBJECT MATTER

*A61C 13/083*(2006.01)i; *C04B 35/488*(2006.01)i
FI:   C04B35/488; A61C13/083

According to International Patent Classification (IPC) or to both national classification and IPC

### B. FIELDS SEARCHED

Minimum documentation searched (classification system followed by classification symbols)

A61C13/083; C04B35/00-C04B35/84

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2020-109069 A (YAMAKIN CO LTD) 16 July 2020 (2020-07-16) | 1-4, 6-17 |
| | claims, paragraphs [0043]-[0045], [0049], [0127]-[0169] | |
| Y | | 5 |
| X | JP 2019-163246 A (SHOFU INC) 26 September 2019 (2019-09-26) | 1-5, 8-9, 12-17 |
| | claims, table 4, paragraphs [0007], [0025], [0046] | |
| Y | US 2016/0120765 A1 (SAGEMAX BIOCERAMICS, INC.) 05 May 2016 (2016-05-05) | 5 |
| | claims, paragraph [0045], fig. 3 | |
| A | WO 2019/131782 A1 (KURARAY NORITAKE DENTAL INC.) 04 July 2019 (2019-07-04) | 1-17 |
| P, X | WO 2022/004862 A1 (KURARAY NORITAKE DENTAL INC.) 06 January 2022 (2022-01-06) | 1-17 |
| | claims | |

☐ Further documents are listed in the continuation of Box C.   ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **01 February 2023** | **14 February 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2022/048500**

| Patent document cited in search report | | | Publication date (day/month/year) | Patent family member(s) | | | Publication date (day/month/year) |
|---|---|---|---|---|---|---|---|
| JP | 2020-109069 | A | 16 July 2020 | (Family: none) | | | |
| JP | 2019-163246 | A | 26 September 2019 | US<br>claims, table 4, paragraphs<br>[0008], [0042], [0073]<br>CN | 2019/0380815<br><br>110304918 | A1<br><br>A | |
| US | 2016/0120765 | A1 | 05 May 2016 | (Family: none) | | | |
| WO | 2019/131782 | A1 | 04 July 2019 | US<br><br>CN | 2021/0061715<br><br>111511702 | A1<br><br>A | |
| WO | 2022/004862 | A1 | 06 January 2022 | JP<br>claims | 2022-60200 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019131782 A
- US 20130221554

- US 20160120765